# EUROPEAN PATENT APPLICATION

(11) **EP 1 113 004 A2**
(43) Date of publication of application: **04.07.2001**
(21) Application number: 01109566.8
(22) Date of filing: 18.03.1998
(51) Int. Cl.: C07C 327/58, C07C 257/22, C07D 409/04, C07D 405/04, C07D 409/14, C07D 413/14, A01N 43/653, C07C 281/04, C07C 311/49

(54) **Intermediates for producing 3-(substituted phenyl)-5-(thienyl or furyl)-1,2,4-triazole compounds**

(30) Priority: 24.04.1997 US 44697 P; 19.11.1997 US 66135 P
(62) Divisional of application: 98914256.7
(71) Applicant: Dow Agrosciences LLC, Indianapolis, Indiana 46268-1054 (US)
(72) Inventor: Pechacek, James T., Indianapolis, IN 46260 (US); Tisdell, Francis E., Carmel, IN 46032 (US); De Vries, Donald H., Fishers, IN 46038 (US); Suhr, Robert G., Greenfield, IN 46140 (US); Johnson, Peter L., Indianapolis, IN 46260 (US); Stockdale, Gary D., Indianapolis, IN 46240 (US); Ash, Mary L., Zionsville, IN 46077 (US); Hamilton, Christopher T., Indianapolis, IN 46234 (US); Hatton, Christopher J., Westfield, IN 46074 (US); Johnson, George W., Indianapolis, IN 46256 (US); Yap, Maurice Chee Hoong, Zionsville, IN 46077 (US)
(74) Representative: Raynor, John

(57) **Abstract**

Novel intermediates of the formula (6) and (7) below are provided, for use in preparing 3-(substituted phenyl)-5-(thienyl or furyl)-1,2,4-triazole compounds, as disclosed in EP-A-0922043. The compounds of EP-A-0922043 that are useful as insecticides and acaricides 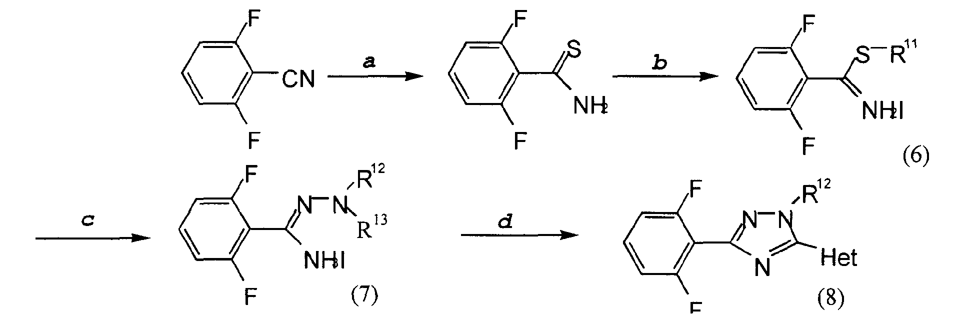
R¹¹ is lower alkyl, preferably methyl;
R¹² is lower alkyl, preferably methyl; R¹³ is a conventional amine protecting group, such as COOt-Bu.

## Description

This invention provides intermediates for producing new compounds that are useful as insecticides and acaricides.

Patent Application No. 98914256.7 (EP-A-0922043) from which this patent application is divided relates to novel substituted thienyl and furanyl triazole derivatives especially useful for the control of insects and mites,
of the formula (1) 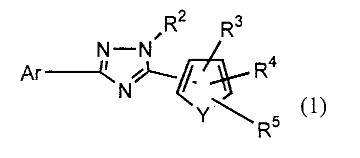 wherein
Ar is substituted phenyl;
Y is O or S;
R² is lower alkyl, haloalkyl, lower alkenyl, lower alkynyl, or alkoxyalkyl;
R³ is selected from H, halo, lower alkyl, (C₇-C₂₁) straight or branched chain alkyl, hydroxy, lower alkoxy, haloalkyl, haloalkoxy, alkoxyalkyl, alkoxyalkoxy, lower alkenyl, lower alkynyl, haloalkenyl, CN, NO₂, COR⁶, CO₂R⁶, CON(R⁶)₂, (C₃-C₆) cycloalkyl, S(O)ₘR⁶, -OSC₂R⁶, SCN, -(CH₂)ₙR⁶, -CH=CHR⁶, -C=OR⁶ - (CH₂)_{q}OR⁶, -(CH₂)_{q}SR⁶ , -(CH₂)_{q}NR⁶R⁶ , -O(CH₂)_{q}R⁶, -S(CH₂)_{q}R⁶, NR⁶(CH₂)_{q}R⁶, 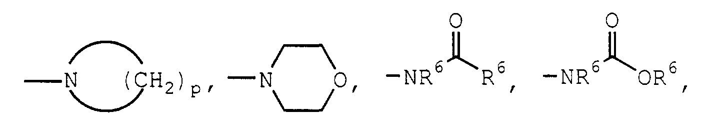 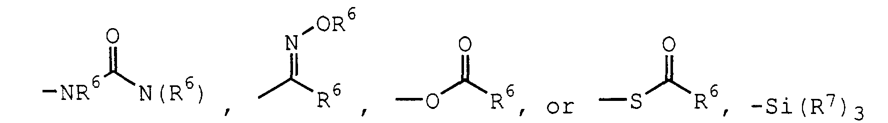 pyridyl, substituted pyridyl, isoxazolyl, substituted isoxazolyl, naphthyl, substituted naphthyl, phenyl, substituted phenyl, thienyl, substituted thienyl, pyrimidyl, substituted pyrimidyl, pyrazolyl, or substituted pyrazolyl;
R⁴ and R⁵ are independently H, halo, lower alkyl, lower alkoxy, haloalkyl, haloalkoxy, CN, CO₂R⁶, CON(R⁶)₂, or S(O)ₘ alkyl, or
if R⁴ and R⁵ are attached to adjacent carbon atoms, they may join to form a 5 or 6 member saturated or unsaturated carbocyclic ring which may be substituted by 1 or 2 halo, lower alkyl, lower alkoxy or haloalkyl groups;
R⁶ is H, lower alkyl, haloalkyl, lower alkenyl, lower alkynyl, phenyl, or substituted phenyl;
R⁷ is lower alkyl;
m is 0, 1, or 2;
n is 1 or 2;
p is an integer from 2 to 6; and
q is 0 or 1;
or a phytologically acceptable acid addition salt thereof.

A particularly preferred class of compounds includes those of formula (1B) 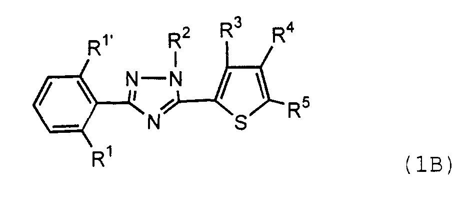 Wherein
R¹ and R¹' are independently F or Cl;
R² is lower alkyl, with methyl being most preferred; and
R³, R⁴ and R⁵ are independently H, Cl, or Br.

Throughout this document, all temperatures are given in degrees Celsius, and all percentages are weight percentages unless otherwise stated.

The term "lower alkyl" refers to (C₁-C₆) straight hydrocarbon chains and (C₃-C₆) branched and cyclic hydrocarbon groups.

The terms "lower alkenyl" and "lower alkynyl" refer to (C₂-C₆) straight hydrocarbon chains and (C₃-C₆) branched hydrocarbon groups containing at least one double or triple bond, respectively.

The term "lower alkoxy" refers to -O-lower alkyl.

The terms "halomethyl" and "haloalkyl" refer to methoxy and lower alkyl groups substituted with one or more halo atoms.

The terms "halomethoxy" and "haloalkoxy" refer to methyl and lower alkoxy groups substituted with one or more halo atoms.

The term "alkoxyalkyl" refers to a lower alkyl group substituted with a lower alkoxy group.

The terms "substituted naphthyl", "substituted thienyl," "substituted pyrimidyl," "substituted pyrazolyl," "substituted pyridyl," and "substituted isoxaxolyl" refer to the ring system substituted with one or more groups independently selected from halo, halo (C₁-C₄) alkyl, CN, NO₂, (C₁-C₄) alkyl, (C₃-C₄) branched alkyl, phenyl, (C₁-C₄) alkoxy, or halo (C₁-C₄) alkoxy.

The term "substituted phenyl" refers to a phenyl group substituted with one or more groups independently selected from halo, (C₁-C₁₀) alkyl, branched (C₃-C₆) alkyl, halo (C₁-C₇) alkyl, hydroxy (C₁-C₇) alkyl, (C₁-C₇) alkoxy, halo (C₁-C₇) alkoxy, phenoxy, phenyl, NO₂, OH, CN, (C₁-C₄) alkanoyl, benzoyl, (C₁-C₄) alkanoyloxy, (C₁-C₄) alkoxycarbonyl, phenoxycarbonyl, or benzoyloxy.

The term "substituted benzenesulfonyl" refers to p-chlorobenzenesulfonyl and p-toluenesulfonyl.

Unless otherwise indicated, when it is stated that a group may be substituted with one or more substituents selected from an identified class, it is intended that the substituents may be independently selected from the class.

### Synthesis

Compounds of formula (1) can be prepared by the methods described in U.S. Patent Nos. 5,380,944 and 5,284,860 (Production Methods 1, 2 and 3). Additional methods will be described hereinafter.

For example, compounds of Formula (1) can be prepared in accordance with the following reaction Scheme I: 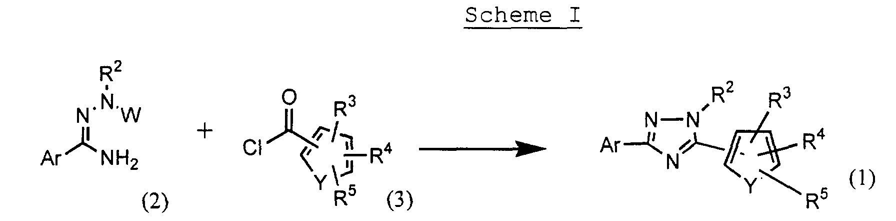 wherein Ar, R², R³, R⁴, R⁵ and Y are as defined in formula (1), and W is a conventional amino protecting group. Examples of conventional amino protecting groups include, but are not limited to, the carbobenzyloxy group, tertiary alkoxycarbonyl groups, amides, phosphinyl and phosphoryl groups, and sulfenyl and sulfonyl groups. As illustrated in Scheme I, an N-protected amidrazone (2) is reacted with a compound of formula (3) in the presence of acid or base as catalyst. Intermediates of formulas (2) and (3) may be obtained by application of well known procedures.

Scheme II illustrates preparation of the protected benzamidrazone starting material (2). 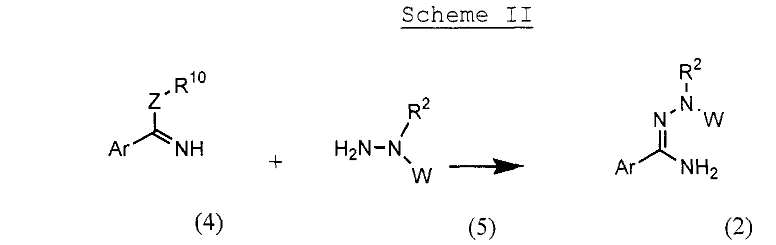 Benzimidate derivative (4), wherein Z is O or S, and R¹⁰ is lower alkyl, is reacted with hydrazine derivative (5), wherein Ar, W, and R² are as defined above for Scheme I.

An example of an intermediate of formula (5) is N-methyl-N-t-butylcarboxyhydrazine. Its use in making regiospecific 1-alkyl [1,2,4] triazoles is found in *Chem.* *Ber.* **1982**, *115,* 2807-2818. The production of benzimide compounds is well known. An example is disclosed in *Synth. Common.* **1983**, *13*, 753.

Another aspect of the invention of EP-A-0922043 is a new method for preparing compounds of formula (1B) wherein R¹ and R¹' are F, as illustrated in Scheme III: 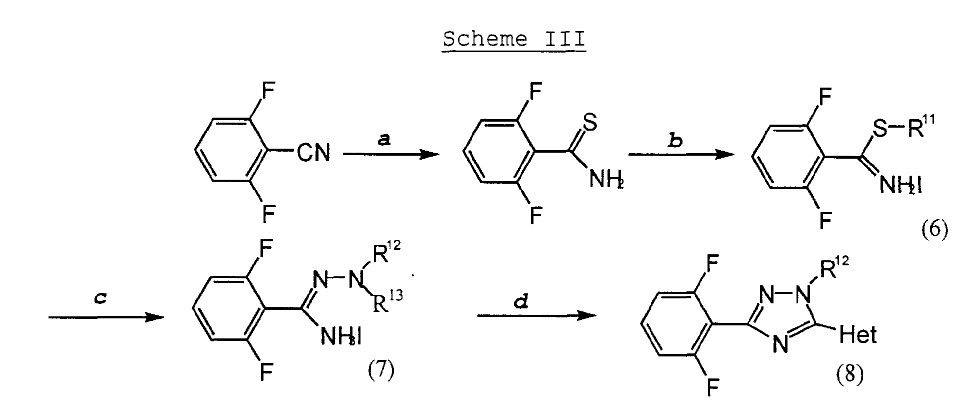 In Scheme III, R¹¹ is lower alkyl, preferably methyl;
R¹² is lower alkyl, preferably methyl; R¹³ is a conventional amine protecting group, such as COOt-Bu; and,
Het is a thienyl or furyl group of formula (9) 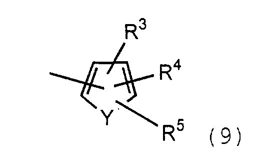 where Y, R³, R⁴, and R⁵ are as defined in formula (1). The process illustrated in Scheme III is also applicable to preparation of compounds wherein Het is any of a variety of other heterocyclic groups, for example pyridyl and pyrazolyl.

The present invention provides novel intermediates of the formulas (6) and (7), as defined above.

Since compounds (6) and (7) are novel, the processes for preparing them are, by definition, also novel. Accordingly the invention also provides a process for preparing a compound of formula (6) above, comprising reacting a compound of formula 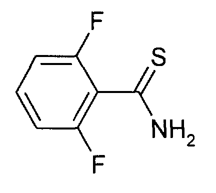 with a lower alkyl iodide in a polar aprotic solvent.

The invention also provides a process for preparing a compound of formula 7, which comprises the steps of
(a) reacting a compound of formula (6) 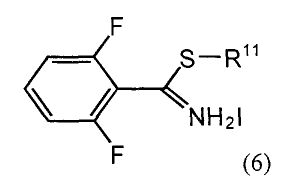 wherein R¹¹ is lower alkyl,
with a compound of formula 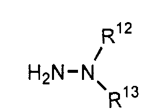 to produce a compound of formula (7) 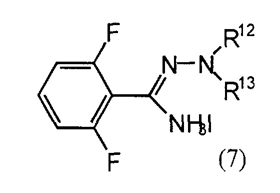 wherein R¹² is lower alkyl, and R¹³ is a conventional amino protecting group.

As illustrated in step ***a*** of Scheme III, 2,6-difiuorobenzonitrile is reacted with triethylamine, sodium sulfide hydrate, and hydrochloric acid in pyridine at room temperature to give 2,6-difluorobenzenethioamide.

In step ***b*** of Scheme III the 2,6-difluorobenzenethioamide is reacted with lower alkyl iodide, e.g. iodomethane, in acetone to provide an S-(lower alkyl)thio-2,6-difluorobenzimidinium iodide of formula (6). Acetone is the preferred solvent, however other polar aprotic solvents such as DMF or THF can be used.

In step ***c*** of Scheme III the S-(lower alkyl)thio-2,6-difluorobenzimidinium iodide is reacted with an N-amino protected-N-(lower alkyl)hydrazine to provide the amidrazone of formula (7). The reaction is carried out in methanol or ethanol, preferably methanol, at a temperature of 0°C to the boiling point of the solvent.

In step ***d*** of Scheme III, the amidrazone of formula (7) is reacted with a thiophene or furan acid chloride in a nonreactive organic solvent such as benzene, toluene, xylenes, chloroform, dichloromethane, or 1,2-dichloroethane, at a temperature in the range from 0°C to the boiling point of the solvent.

The process of Scheme III uses milder conditions than previously published processes, and therefore allows thermally sensitive heterocycles to be used. Higher yields are also provided.

A detailed illustration of steps ***a-c*** of Scheme III is given in Example 1 hereinafter. Detailed illustrations for step ***d*** are given in EP-A-0922043.

### Example 1

The following steps illustrate preparation of the amidrazone of formula (7a)

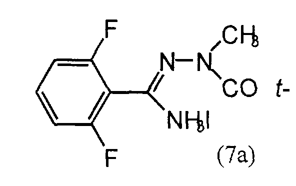

### A. 2, 6-difluorobenzenethioamide.

Into a 3 L three necked round bottom flask equipped with a mechanical stirrer, dry ice condenser, dropping funnel, and outlet to a trap filled with bleach was added pyridine (550 mL), 2,6-difluorobenzonitrile (208 g, 1.50 mol), triethylamine (202 g, 279 mL, 2.0 mol), and sodium sulfide hydrate (521 g, 2.17 mol-broken into pieces small enough to fit into the flask). The temperature of the stirred mixture was lowered to approximately 5 °C and to the slurry was added dropwise concentrated hydrochloric acid (143 g, 288 mL, 3.99 mol). An exotherm was noted and the rate of addition of the hydrochloric acid was such that the temperature of the reaction mixture did not exceed 25 °C for a total addition time of 75 min. The cooling bath was removed and the slurry was allowed to warm to RT and to stir over night. The mixture was poured into water (2 L) and was extracted with ether (3 X 500 mL). The ether layer was washed with dilute sulfuric acid, water, brine, dried (MgSO₄), and the solvent removed *in vacuo* to give 232 grams of crude product. The starting material was removed from the product via kugelrohr distillation to give 197 g (76%) of 2,6-difluorobenzenethioamide. This material was used without further purification.

### B. S-methylthio-2,6-difluorobenzamidinium iodide

Into a 3 L three necked flask equipped with a mechanical stirrer and dropping funnel was added acetone (1150 mL) and 2,6-difluorobenzenethioamide (197 g, 1.14 mol). The temperature of the stirred solution was lowered to approximately 5 °C and iodomethane (161 g, 70.6 mL, 1.14 mol) was added dropwise. The ice bath was removed and the slurry was allowed to stir over night. The resulting yellow solids were removed via filtration and washed with ether to obtain 223 grams. An additional portion of material was obtained from the filtrate by removal of the solvent *in vacuo*. Ether was added to the residue and the resulting solids removed via filtration to obtain an additional 57 grams of material. The combined solids totaled 280 g (77.9% yield) of S-methylthio-2,6-difluoro-benzimidinium iodide: mp 168-169 °C; ¹H NMR (DMSO-d₆) δ 7.7 (m, 1H) , 7.4 (m, 2H), 2.7 (s, 3H).

### C. N-tert-butoxycarbonyl-N-methylhydrazine

Into a 1 L three necked round bottom flask equipped with a mechanical stirrer and dropping funnel was added methyl hydrazine (42.2 g, 0.916 mol) and THF (100 mL). The temperature of the mixture was cooled to 5 °C and a solution of di-tert-butyl dicarbonate (100 g, 0.458 mol) dissolved in THF (150 mL) was added dropwise. The cooling bath was removed and the mixture was stirred at RT overnight. The liquid was decanted from a gummy precipitate and the solvent removed *in vacuo* to give approximately 70 grams of a clear liquid. The gummy precipitate was partitioned between methylene chloride and water. The methylene chloride was washed with brine, dried (Na₂SO₄) and the solvent removed *in vacuo*. The resulting residue was combined with that from the previous evaporation and distilled at approximately 20 mm Hg (bp 77-78 °C) to give 40.2 g (60% yield) of N-tert-butoxycarbonyl-N-methylhydrazine: ¹H NMR (CDCl₃) δ 4.1 (s, b, 2H), 3.05 (s, 3H), 1.5 (s, 9H).

### D. Amidrazone of formula (7a)

Into a 1 L round bottom flask equipped with a mechanical stirrer, dropping funnel, and outlet to a trap filled with bleach, was added S-methyl-2,6-difluorobenziminium iodide (63.8 g, 0.202 mol) and methanol (180 mL). To the stirred solution was added dropwise N-tert-butoxycarbonyl-N-methylhydrazine (29.6 g. 0.202 mol). The solution was allowed to stir overnight and the methanol was removed *in vacuo.* The residue was triturated with ether and the solids removed via filtration to give 66.3 grams (79.0% yield) of the amidrazone of formula (7a): mp 172-173 °C (dec); ¹H NMR (DMSO-d₆) δ 12.3 (s, b, 1H) , 10.4 (d, b, 2H), 7.9 (m, 1H) , 7.4 (m, 2H), 3.1 (s, 3H), 1.5 (s, 9H).

The use of compounds (6) and (7) in the preparation of compounds of formula (1) is fully described in EP-A-0922043.

## Claims

1. A compound of the formula (6) 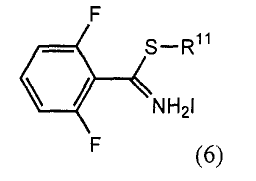 wherein R¹¹ is lower alkyl.

2. A compound of the formula (7b) 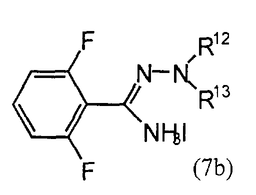 wherein R¹² is lower alkyl, and R¹³ is a conventional amino protecting group.

3. A compound of claim 2, wherein R¹³ is COOt-Bu.

4. A process for preparing a compound of formula (6) as claimed in claim 1, which comprises reacting a compound of formula 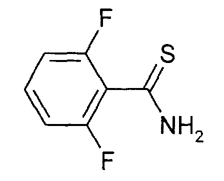 with a lower alkyl iodide in a polar aprotic solvent.

5. A process as claimed in claim 4, wherein the solvent is acetone, DMF or THF.

6. A process for preparing a compound of formula (2b) as defined in claim 4 which comprises the steps of
(a) reacting a compound of formula (6) 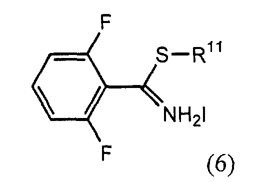 wherein R¹¹ is lower alkyl,
with a compound of formula 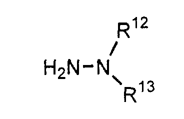 to produce a compound of formula (7) 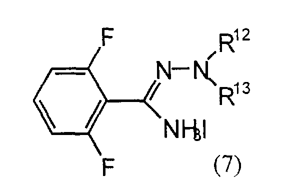 wherein R¹² is lower alkyl, and R¹³ is a conventional amino protecting group.
